# EUROPÄISCHE PATENTANMELDUNG

(11) **EP 4 008 393 A1**
(43) Veröffentlichungstag der Anmeldung: **08.06.2022**
(21) Anmeldenummer: 20211949.1
(22) Anmeldetag: 04.12.2020
(51) Int. Cl.: A61M 60/122, A61M 60/205, A61M 60/592, A61M 60/871, A61M 60/88

(54) **KONNEKTOREINHEIT, STEUEREINHEIT UND STEUERSYSTEM**

(71) Anmelder: Berlin Heart GmbH, 12247 Berlin (DE)
(72) Erfinder: FRISCHKE, Michael, 15834 Rangsdorf (DE)
(74) Vertreter: Pfenning, Meinig & Partner mbB

(57) **Zusammenfassung**

Die Anmeldung betrifft eine Konnektoreinheit (3) für eine zum Ansteuern einer implantierbaren Blutpumpe (4) eingerichtete Steuereinheit (2), wobei die Konnektoreinheit (3) umfasst: eine Versorgungsschnittstelle (5), eingerichtet zum Verbinden der Konnektoreinheit (3) mit einer Stromversorgung (6), eine Datenschnittstelle (7), eingerichtet zum Verbinden der Konnektoreinheit (3) mit einer Datenverarbeitungseinheit (8), und eine Steuerschnittstelle (9), eingerichtet zum Verbinden der Konnektoreinheit (3) mit einer integrierten Schnittstelle (10) der Steuereinheit (2), so dass zugleich eine Versorgungsverbindung der Steuereinheit (2) mit der Stromversorgung (6) und eine Datenverbindung der Steuereinheit (2) mit der Datenverarbeitungseinheit (8) mittels der Konnektoreinheit (3) herstellbar sind. Die Anmeldung betrifft ferner eine Steuereinheit (2) zum Ansteuern einer implantierbaren Blutpumpe (4) sowie ein Steuersystem für eine implantierbare Blutpumpe (4).

## Beschreibung

Die Anmeldung betrifft eine Konnektoreinheit für eine zum Ansteuern einer implantierbaren Blutpumpe eingerichtete Steuereinheit, eine Steuereinheit zum Ansteuern einer implantierbaren Blutpumpe sowie ein Steuersystem für eine implantierbare Blutpumpe.

Steuersysteme für implantierbare Blutpumpen und Komponenten solcher Steuersysteme, insbesondere Steuereinheiten und Konnektoreinheiten, sind aus dem Stand der Technik bekannt. Bei Steuersystemen gemäß dem Stand der Technik ist die Steuereinheit häufig mittels einer Kabelverbindung mit einem externen Akku zur Spannungsversorgung verbunden.

Ein solches Steuersystem kann hinsichtlich seiner Kompaktheit, Robustheit, Handhabung und Standzeit verbesserungsbedürftig sein.

Bei einer Operation zum Implantieren einer Blutpumpe ist es ferner vorteilhaft, die Steuereinheit innerhalb eines sterilen Operationsbereichs zum Verbinden mit der Blutpumpe bereitzustellen, weshalb das Steuersystem und seine Komponenten für eine solche Verwendung ausgelegt sein sollten.

Dementsprechend soll die Steuereinheit im Operationsbereich mit Spannung versorgbar sowie extern ansteuerbar und/oder programmierbar sein. Es können jedoch keine unsterilen Objekte (etwa Kabelverbindungen oder sonstige Konnektoreinheiten, externe Datenverarbeitungseinheiten oder externe Ackus, soweit sie nicht steril bzw. auf praktikable Weise sterilisierbar sind) in den sterilen Operationsbereich eingebracht werden.

Weiterhin ist bei medizinischen Geräten ein Sicherer Betrieb, insbesondere eine elektrische Sicherheit, von großer Bedeutung.

Vor dem Hintergrund des Stands der Technik liegt der vorliegenden Anmeldung die Aufgabe zugrunde, eine Konnektoreinheit, eine Steuereinheit sowie ein Steuersystem dergestalt vorzuschlagen, dass eine Spannungsversorgung und/oder Ansteuerung der Steuereinheit innerhalb eines sterilen Operationsbereichs auf möglichst einfache Weise erfolgen kann, wobei das die Konnektoreinheit, die Steuereinheit und das Steuersystem möglichst kompakt, robust, einfach zu handhaben und langlebig sein sollen und einen sicheren Betrieb ermöglichen.

Zur Lösung der Aufgabe werden eine Konnektoreinheit gemäß Anspruch 1, eine Steuereinheit gemäß Anspruch 7 und ein Steuersystem gemäß Anspruch 13 vorgeschlagen. Vorteilhafte Ausgestaltungen und Weiterentwicklungen ergeben sich mit den Merkmalen gemäß den Unteransprüchen.

Die vorgeschlagene Konnektoreinheit für eine zum Ansteuern einer implantierbaren Blutpumpe eingerichtete Steuereinheit umfasst:
eine Versorgungsschnittstelle, eingerichtet zum Verbinden der Konnektoreinheit mit einer Stromversorgung,
eine Datenschnittstelle, eingerichtet zum Verbinden der Konnektoreinheit mit einer Datenverarbeitungseinheit,
eine Steuerschnittstelle, eingerichtet zum Verbinden der Konnektoreinheit mit einer integrierten Schnittstelle der Steuereinheit, so dass zugleich eine Versorgungsverbindung der Steuereinheit mit der Stromversorgung und eine Datenverbindung der Steuereinheit mit der Datenverarbeitungseinheit mittels der Konnektoreinheit herstellbar sind.

Mittels einer solchen Konnektoreinheit ist es möglich, eine entsprechende Steuereinheit in einem sterilen Operationsbereich mit Spannung zu versorgen und/oder extern anzusteuern und/oder extern zu programmieren, indem die beschriebene Versorgungsverbindung und/oder die beschriebene Datenverbindung hergestellt wird.

Mittels der Versorgungsverbindung kann die Steuereinheit dann mit Spannung versorgt werden, mittels der Datenverbindung kann die Steuereinheit extern angesteuert und/oder programmiert werden.

Dabei ist zwischen der Steuerschnittstelle der Konnektoreinheit und der integrierten Schnittstelle der Steuereinheit nur eine sterile Kabelverbindung erforderlich, wodurch der Einsatz im sterilen Operationsbereich auf einfache und sichere Weise ermöglicht wird. Kabelverbindungen zwischen der Konnektoreinheit und der Stromversorgung und/oder der Datenverarbeitungseinrichtung sowie die Stromversorgung und/oder Datenverarbeitungseinrichtung selbst können unsteril sein und außerhalb des sterilen Operationsbereichs angeordnet sein.

Die Konnektoreinheit und/oder das Verbindungskabel kann insbesondere sterilisierbar, etwa chemisch und/oder durch Bestrahlung und/oder durch Druck- und/oder Temperatureinwirkung sterilisierbar, sein. Materialien und/oder Verarbeitung (beispielsweise Versiegelungen und/oder Dichtungen) der Konnektoreinheit können zum Gewährleisten der Sterilisierbarkeit ausgelegt sein.

Die Konnektoreinheit kann aufgrund der gemeinsam ausgeführten Versorgungs- und Datenverbindung kompakt ausgebildet sein und somit ein kompaktes Steuersystem ermöglichen. Aufgrund der geringen Anzahl von Verbindungen wird zudem die elektrische Sicherheit erhöht und die Handhabung verbessert.

Sterilisierbare Komponenten, z. Bsp. die Steuereinheit und/oder die Konnektoreinheit und/oder das Verbindungskabel, können sich optisch von nicht sterilisierbaren und damit immer unsterilen Komponenten unterscheiden. Hierfür kommen z. Bsp Gehäusefarbe und/oder ein entsprechender Aufdruck in Frage. An der Konnektoreinheit kann der Übergang zu den nicht sterilisierbaren bzw. unsterilen Komponenten gesondert markiert sein.

Ferner kann eine Steuereinheit, die zur Verwendung mit der Konnektoreinheit eingerichtet ist, besonders kompakt, robust, einfach handhabbar und langlebig ausgeführt sein, da allein mittels des integrierten Schnittstelle die Datenverbindung und die Versorgungsverbindung herstellbar sind, also keine zusätzliche Daten- oder Versorgungsschnittstelle erforderlich ist.

Die Konnektoreinheit kann dazu eingerichtet sein, mit der Steuereinheit mittels der integrierten Schnittstelle und der Steuerschnittstelle unter Verwendung eines ersten Kommunikationsprotokolls zu kommunizieren. Die Konnektoreinheit kann dazu eingerichtet sein, mit der Datenverarbeitungseinheit mittels der Datenschnittstelle unter Verwendung eines zweiten Kommunikationsprotokolls eingerichtet sein. Die Konnektoreinheit kann zur Umwandlung von Kommunikationsdaten zwischen dem ersten und zweiten Kommunikationsprotokoll eingerichtet sein. Das erste und/oder zweite Kommunikationsprotokoll kann beispielweise ein Protokoll aus der Gruppe USB, UART, CAN, RS485, SPI oder ein sonstiges serielles oder paralleles Kommunikationsprotokoll sein.

Als Stromversorgung kann ein Netzgerät und/oder eine Steckdose und/oder ein Akku und/oder eine andere Art von Stromversorgung vorgesehen sein. Als Datenverarbeitungseinheit kann ein Workstation-Rechner, ein tragbares Endgerät oder eine andere Art von Datenverarbeitungseinheit zum Ansteuern und/oder Programmieren der Steuereinheit vorgesehen sein.

Die Versorgungsschnittstelle, die Datenschnittstelle und die Steuerschnittstelle sind vorzugsweise drahtgebundene elektrische Schnittstellen. Als drahtgebunden wird hier eine elektrische Schnittstelle bezeichnet, mittels derer ein direkter elektrisch leitfähiger Kontakt zwischen zwei Komponenten herstellbar ist.

Die Konnektoreinheit kann ein Gehäuse und mindestens eine Leiterplatte und/oder integrierte Schaltung umfassen. Die Konnektoreinheit kann alternativ beispielsweise als Y-Kabel ohne eigene Schaltungen gebildet sein.

Die Versorgungsschnittstelle kann einen Konnektor zum Herstellen einer lösbaren Kabelverbindung mit der Stromversorgung aufweisen. Die Datenschnittstelle kann einen Konnektor zum Herstellen einer lösbaren Kabelverbindung mit der Datenverarbeitungseinheit aufweisen. Die Steuerschnittstelle kann einen Konnektor zum Herstellen einer lösbaren Kabelverbindung mit der Steuereinheit aufweisen. Die Versorgungsschnittstelle und/oder die Datenschnittstelle und/oder die Steuerschnittstelle kann mit einem Verbindungskabel fest verbunden sein.

Die Konnektoreinheit kann insbesondere ein Verbindungskabel umfassen, wobei die Steuerschnittstelle fest oder lösbar mit einem ersten Ende des Verbindungskabels verbindbar oder verbunden ist und ein zweites Ende des Verbindungskabels einen mit der integrierten Schnittstelle der Steuereinheit zum Herstellen der Versorgungsverbindung und der Stromverbindung verbindbaren integrierten Konnektor aufweist.

Die Konnektoreinheit kann wasserdicht sein. Der integrierte Konnektor des Verbindungskabels kann wasserdicht mit der integrierten Schnittstelle der Steuereinheit verbindbar sein. So können ein sicherer Betrieb sowie eine einfache Reinigung der Konnektoreinheit ermöglicht werden.

Die Datenschnittstelle kann eine USB-Schnittstelle sein oder umfassen. Die Konnektoreinheit kann eine Schnittstellen-Controller-Schaltung, insbesondere eine USB-Controller-Schaltung, umfassen. So kann eine Datenschnittstelle für eine Operation und/oder eine Wartung und/oder Bedienung der Steuereinheit durch medizinisches Personal zur Verfügung gestellt werden, selbst wenn die Steuereinheit selbst nicht alle dafür notwendige Schnittstellenlogik umfasst.

Auch kann so vermieden werden, zusätzliche Schnittstellenlogik in der Steuereinheit vorzusehen, die möglicherweise nur während einer Operation und/oder eine Wartung und/oder Bedienung der Steuereinheit durch medizinisches Personal, nicht aber im regulären Betrieb beim Patienten benötigt wird.

Die Konnektoreinheit kann, insbesondere zusätzlich zu einer drahtgebundenen Datenschnittstelle, eine drahtlose Datenschnittstelle, beispielsweise eine Bluetooth-Schnittstelle, zum Herstellen einer drahtlosen Datenverbindung mit einer Datenverarbeitungseinheit umfassen. Somit kann die Konnektoreinheit ein besonders flexibles Ansteuern und/oder Programmieren der Steuereinheit ermöglichen.

Die Konnektoreinheit kann eine Isolationsbarriere, insbesondere eine Luftstrecke und/oder Kriechstrecke, zum elektrischen Entkoppeln der Steuereinheit von der Datenverarbeitungseinheit umfassen. Durch das elektrische Entkoppeln mittels der Isolationsbarriere kann die elektrische Sicherheit der Konnektoreinheit und/oder des Steuersystems gewährleistbar sein. Insbesondere kann vorgesehen sein, dass die Isolationsbarriere den Anforderungen einer Norm für die Sicherheit medizinischer Geräte, insbesondere der Norm IEC 60601-1 in der zum Anmeldetag geltenden Fassung, genügt.

Die vorgeschlagene Steuereinheit zum Ansteuern einer implantierbaren Blutpumpe umfasst eine integrierte Schnittstelle, die mit der Steuerschnittstelle einer Konnektoreinheit der vorgeschlagenen Art zum Herstellen der Versorgungsverbindung und der Datenverbindung verbindbar ist.

Wie oben erläutert, kann die auf diese Weise zur Verwendung mit der Konnektoreinheit eingerichtete Steuereinheit, kompakt, robust und langlebig ausgeführt sein und eignet sich für einen Betrieb im sterilen Operationsbereich.

Die Steuereinheit kann insbesondere sterilisierbar etwa chemisch und/oder durch Bestrahlung und/oder durch Druck- und/oder Temperatureinwirkung sterilisierbar, sein. Materialien und/oder Verarbeitung (beispielsweise Versiegelungen und/oder Dichtungen) der Steuereinheit können zum Gewährleisten der Sterilisierbarkeit ausgelegt sein.

Die Steuereinheit kann zum einteiligen Verbinden mit mindestens einem externen Akku eingerichtet sein, d. h. zum Verbinden mit dem mindestens einen externen Akku ohne zwischengeschaltete Kabelverbindung, insbesondere zum formschlüssigen und/oder kraftschlüssigen und/oder wasserdichten einteiligen Verbinden mit dem mindestens einen externen Akku. Durch die einteilige Verbindung mit dem mindestens einen externen Akku kann die Steuereinheit besonders kompakt, robust, einfach handhabbar und langlebig konstruiert werden. Die Steuereinheit kann beispielsweise zum gleichzeitigen einteiligen Verbinden mit zwei externen Akkus eingerichtet sein. Damit kann auch bei Ausfall/Austausch eines externen Akkus die Stromversorgung sichergestellt werden.

Es kann vorgesehen sein, dass an der Steuereinheit angeordnete Kontakte zum Verbinden mit dem externen Akku spannungsfrei schaltbar und/oder elektrisch isolierbar sind. Damit kann die Betriebssicherheit der Steuereinheit in einem Betrieb ohne externen Akku (insbesondere in einem Betrieb mit einem internen Akku und/oder mit einer Netzspannungsversorgung) verbessert werden.

Die Steuereinheit kann eine extrakorporale (also nicht implantierbare) Steuereinheit sein. Die Steuereinheit kann zum Ansteuern der Blutpumpe mittels einer Driveline, die eine reversibel verbindbare Kupplung umfassen kann, mit der Blutpumpe verbindbar sein. Dadurch kann eine Inbetriebnahme (beispielsweise während einer Operation zum Implantieren der Blutpumpe) und/oder ein Ersatz und/oder eine Wartung der Steuereinheit erleichtert werden. Die Steuereinheit kann mit einem TET-Modul (Transcutaneous Energy Transfer-Modul) und/oder einer Übertragungsspule für drahtlose Energieübertragung verbindbar und/oder verbunden sein. Die Blutpumpe kann mittels des TET-Moduls und/oder der Übertragungsspule ansteuerbar sein.

Die Steuereinheit kann einen internen Akku aufweisen. Somit kann die Steuereinheit wenigstens zeitweise betrieben werden, wenn der externe Akku bzw. die externen Akkus und/oder eine externe Stromversorgung ausfallen und/oder nicht verfügbar sind.

Es kann vorgesehen sein, dass die Steuereinheit außer den folgenden vier externen drahtgebundenen elektrischen Schnittstellen keine weitere externe drahtgebundene elektrische Schnittstelle aufweist: die vorgenannte integrierte Schnittstelle, eine Driveline-Schnittstelle zum Verbinden der Steuereinheit mit der Blutpumpe, eine Akkuschnittstelle zum Verbinden der Steuereinheit mit einem externen Akku und entweder eine verschließbare, vorzugsweise wasserdicht verschließbare, Wartungsschnittstelle zum Einsetzen eines internen Akkus oder eine zusätzliche Akkuschnittstelle zum Verbinden der Steuereinheit mit einem zusätzlichen externen Akku. Es kann vorgesehen sein, dass die Steuereinheit sowohl die Wartungsschnittstelle als auch die zusätzliche Akkuschnittstelle aufweist und außer den somit gegebenen fünf externen drahtgebundenen elektrischen Schnittstellen keine weitere externe drahtgebundene elektrische Schnittstelle aufweist.

Dass so eine minimale Anzahl von drahtgebundenen elektrischen Schnittstellen vorgesehen ist, ist besonders vorteilhaft für die elektrische Sicherheit und/oder Dichtigkeit (vgl. für elektrische Betriebsmittel relevante IP-Schutzklassen).

Das vorgeschlagene Steuersystem für eine implantierbare Blutpumpe umfasst eine Konnektoreinheit der vorgeschlagenen Art und eine Steuereinheit der vorgeschlagenen Art. Bestimmte Aspekte und Vorteile des Steuersystems ergeben sich etwa aus den vorgenannten Aspekten und Vorteilen der Konnektoreinheit und der Steuereinheit.

Das Steuersystem kann eine Stromversorgungseinheit umfassen, die wahlweise direkt oder mittels der Konnektoreinheit mit der integrierten Schnittstelle der Steuereinheit verbindbar ist. Damit kann die Stromversorgungseinheit etwa vorteilhaft während einer Operation und/oder einer Wartung und/oder Bedienung der Steuereinheit durch medizinisches Personal mittels der Konnektoreinheit verbunden sein, womit gleichzeitig eine Datenverbindung herstellbar ist, im regulären Betrieb beim Patienten dagegen direkt mit der Steuereinheit verbunden sein. Die Stromversorgungseinheit kann zum netzgebundenen Betreiben der Steuereinheit und/oder zum Laden des externen und/oder internen Akkus eingerichtet sein. Die Stromversorgungseinheit kann selbst ein Akku sein oder einen Akku umfassen.

Die Steuereinheit des Steuersystems kann gemeinsam mit der Konnektoreinheit des Steuersystems in einer Sterilverpackung versiegelt sein. Somit kann das Steuersystem in einem Zustand bereitstellbar und/oder lagerbar sein, der einen schnellen und einfachen Einsatz etwa im sterilen Operationsbereich bei einer Operation zum Implantieren der Blutpumpe ermöglicht. Zur Lagerung kann die Steuereinheit ohne den externen Akku und/oder den internen Akku in der Sterilverpackung versiegelt sein. Alternativ oder zusätzlich können die Steuereinheit und/oder Teile der Steuereinheit, insbesondere die drahtgebundenen elektrischen Schnittstellen der Steuereinheit, mit einer sterilen Abdeckung, beispielsweise einer Schutzfolie, bedeckt oder bedeckbar sein. Somit können die entsprechenden Teile der Steuereinheit während einer Operation vor Flüssigkeiten und/oder Verschmutzung geschützt werden. Nach der Operation kann die Abdeckung entfernt werden.

Die vorgenannten und weitere Aspekte und Vorteile der vorgeschlagenen Konnektoreinheit, der vorgeschlagenen Steuereinheit und des vorgeschlagenen Steuersystems werden nachfolgend anhand von Zeichnungen, Fig. 1 bis Fig. 3, näher erläutert. Dabei zeigen, jeweils schematisch
Fig. 1 ein Steuersystem für eine implantierbare Blutpumpe,
Fig. 2 ein Blockschaltbild des Steuersystems nach einem weiteren Beispiel,
Fig. 3 eine Schaltskizze eines Teils des Steuersystems nach einem weiteren Beispiel.

Wiederkehrende und ähnliche Merkmale sind in den Zeichnungen mit identischen oder ähnlichen alphanumerischen Bezugszeichen versehen. Bezugszeichen bereits in einer zuvor erläuterten Zeichnung gezeigter Merkmale werden dabei teilweise ausgelassen. Ferner werden bereits im Zusammenhang mit einer zuvor erläuterten Zeichnung erläuterte Bezugszeichen teilweise nicht erneut erläutert.

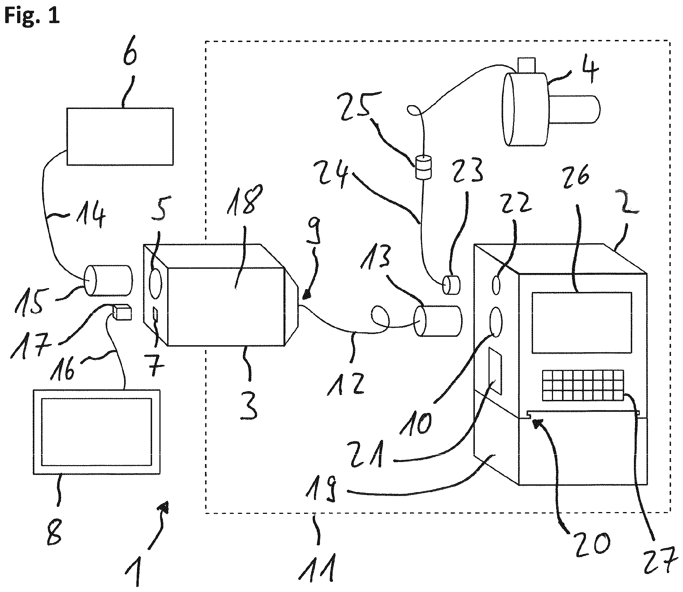

Das in Fig. 1 gezeigte Steuersystem 1 umfasst eine Steuereinheit 2 und eine Konnektoreinheit 3. Die Steuereinheit 2 ist zum Ansteuern einer implantierbaren Blutpumpe 4 eingerichtet (die Blutpumpe 4 selbst ist nicht notwendigerweise Teil des Steuersystems 1).

Die Konnektoreinheit 3 umfasst eine Versorgungsschnittstelle 5, eingerichtet zum Verbinden der Konnektoreinheit 4 mit einer Stromversorgung 6, und eine Datenschnittstelle 7, eingerichtet zum Verbinden der Konnektoreinheit 4 mit einer Datenverarbeitungseinheit 8.

Ferner umfasst die Konnektoreinheit 3 eine Steuerschnittstelle 9, eingerichtet zum Verbinden der Konnektoreinheit 3 mit einer integrierten Schnittstelle 10 der Steuereinheit 2, so dass zugleich eine Versorgungsverbindung der Steuereinheit 2 mit der Stromversorgung 6 und eine Datenverbindung 7 der Steuereinheit 2 mit der Datenverarbeitungseinheit 8 mittels der Konnektoreinheit 3 herstellbar sind.

Mittels der Konnektoreinheit 3 ist es möglich, die Steuereinheit 2 in einem sterilen Operationsbereich 11 mit Spannung zu versorgen und/oder extern anzusteuern und/oder extern zu programmieren, indem die Versorgungsverbindung und/oder die Datenverbindung hergestellt wird. Mittels der Versorgungsverbindung kann die Steuereinheit 2 dabei mit Spannung versorgt werden, mittels der Datenverbindung kann die Steuereinheit 2 extern angesteuert und/oder programmiert werden.

Die Steuerschnittstelle 9 der Konnektoreinheit 3 ist mit der integrierten Schnittstelle 10 der Steuereinheit 2 mittels eines ersten Verbindungskabels 12, umfassend einen ersten Konnektor 13 (integrierter Konnektor, indem die Funktion eines Datenkonnektors und die Funktions eines Konnektors zur Spannungsversorgung integriert sind) verbindbar.

Die Versorgungsschnittstelle 5 ist mit der Stromversorgung 6 mittels eines zweiten Verbindungskabels 14, umfassend einen zweiten Konnektor 15, verbindbar. Die Datenschnittstelle 7 ist mit der Datenverarbeitungseinheit 8 mittels eines dritten Verbindungskabels 16, umfassend einen dritten Konnektor 17, verbindbar.

In der in Fig. 1 illustrierten Konfiguration, die zur Verwendung der Steuereinheit 2 während einer Operation zur Implantation der Blutpumpe 4 geeignet ist, sind die Steuereinheit 2 und das erste Verbindungskabel 12 im sterilen Operationsbereich 11 angeordnet. Die Stromversorgung 6, die Datenverarbeitungseinheit 8, das zweite Verbindungskabel 14 und das dritte Verbindungskabel 16 sind außerhalb des sterilen Operationsbereichs 11 angeordnet. Die Konnektoreinheit 13 ist an einem Übergang zum sterilen Operationsbereich 11 (beispielsweise an einer Sterilbarriere) angeordnet.

Die Konnektoreinheit 3, das erste Verbindungskabel 12 und die Steuereinheit 2 können sterilisierbar, etwa chemisch und/oder durch Bestrahlung und/oder durch Druck- und/oder Temperatureinwirkung sterilisierbar, sein. Die Konnektoreinheit 3 kann ferner wasserdicht sein, der erste Konnektor 13 kann wasserdicht mit der integrierten Schnittstelle 10 der Steuereinheit 2 verbindbar sein.

Als Stromversorgung 6 kann ein Netzgerät oder eine Steckdose oder eine andere Art von Stromversorgung vorgesehen sein. Als Datenverarbeitungseinheit 8 kann ein Workstation-Rechner, ein tragbares Endgerät oder eine andere Art von Datenverarbeitungseinheit zum Ansteuern und/oder Programmieren der Steuereinheit 2 vorgesehen sein.

Die Versorgungsschnittstelle 5, die Datenschnittstelle 7 und die Steuerschnittstelle 9 sind drahtgebundene elektrische Schnittstellen. Die Konnektoreinheit 3 umfasst ein Gehäuse 18, dass eine mit den Schnittstellen verbundene Platine (im Inneren des Gehäuses 18) enthält. Die Konnektoreinheit 3 kann alternativ beispielsweise als Y-Kabel ohne Gehäuse und/oder eigene Schaltungen gebildet sein. Die Datenschnittstelle 7 und/oder die Steuerschnittstelle 9 können alternativ oder zusätzlich drahtlose Schnittstellenkomponenten umfassen.

Die Versorgungsschnittstelle 5 weist einen Konnektor zum Herstellen einer lösbaren Kabelverbindung mit der Stromversorgung auf. Die Datenschnittstelle 7 weist einen Konnektor zum Herstellen einer lösbaren Kabelverbindung mit der Datenverarbeitungseinheit 8 auf. Die Versorgungsschnittstelle 5 und/oder die Datenschnittstelle 7 kann alternativ mit dem jeweiligen Verbindungskabel 14, 16 fest verbunden sein.

Die Steuerschnittstelle 9 ist mit dem ersten Verbindungskabel 12 fest verbunden. Die Steuerschnittstelle 9 kann alternativ einen Konnektor zum Herstellen einer lösbaren Kabelverbindung mit der Steuereinheit 2 aufweisen.

Das Steuersystem 1 umfasst ferner einen externen Akku 19. Die Steuereinheit 2 ist zum einteiligen Verbinden mit dem externen Akku 19 mittels einer Akkuschnittstelle 28 eingerichtet, d. h. zum Verbinden mit dem externen Akku 19 ohne zwischengeschaltete Kabelverbindung. Der externe Akku 19 ist mit der Steuereinheit 2 zum formschlüssigen, kraftschlüssigen (durch Einrasten) und wasserdichten einteiligen Verbinden mit der Steuereinheit 2 mittels einer einrastbaren Einschubschiene 20 eingerichtet. Der externe Akku 19 kann mit der Steuereinheit 2 alternativ mittels einer Kabelverbindung verbindbar sein.

Die Steuereinheit 2 umfasst eine Wartungsschnittstelle 21 zum Einsetzen eines internen Akkus. Die Wartungsschnittstelle 21 ist bevorzugt verschließbar, besonders bevorzugt wasserdicht verschließbar.

Die Steuereinheit 2 ist eine extrakorporale (also nicht implantierbare) Steuereinheit. Die Steuereinheit 2 umfasst eine Driveline-Schnittstelle 22. Die Driveline-Schnittstelle 22 ist mittels einer Driveline 24 (mit einem vierten Konnektor 23) zum Ansteuern der Blutpumpe 4 mit der Blutpumpe 4 verbindbar. Die Driveline 24 umfasst eine reversibel verbindbare Kupplung 25. Alternativ kann die Driveline 24 einteilig, also ohne Kupplung, ausgeführt sein.

Die Steuereinheit 2 umfasst eine Anzeige 26 zum Anzeigen von Betriebsparametern und/oder Steueroptionen der Steuereinheit 2 und/oder der Blutpumpe 4 sowie ein Bedienfeld 27 zum Bedienen der Steuereinheit 2. Das Bedienfeld 27 kann beispielsweise ein Tastenfeld oder ein Touchpanel sein. Das Bedienfeld 27 kann auch in die Anzeige 26 integriert sein, beispielsweise in Verbindung mit einem Touchscreen als Anzeige 26. Die Anzeige 26 und/oder das Bedienfeld 27 kann auch weggelassen werden. In diesem Fall kann die Steuereinheit 2 beispielsweise mit einer externen Anzeige und/oder einer externen Bedieneinheit verbindbar sein

Außer den folgenden vier externen drahtgebundenen elektrischen Schnittstellen umfasst die Steuereinheit 2 keine weitere externe drahtgebundene elektrische Schnittstelle: die integrierte Schnittstelle 10, die Driveline-Schnittstelle 22, die Akkuschnittstelle 28 und die Wartungsschnittstelle 21. Alternativ kann die Steuereinheit 2 mindestens eine weitere drahtgebundene elektrische Schnittstelle umfassen, beispielsweise eine zusätzliche Datenschnittstelle (etwa zum Verbinden mit einer externen Anzeige und/oder einer externen Bedieneinheit).

Die Stromversorgung 6 ist eine Stromversorgungseinheit, die wahlweise direkt oder mittels der Konnektoreinheit 3 mit der integrierten Schnittstelle der Steuereinheit 2 verbindbar ist. Dazu ist der zweite Konnektor 15 wahlweise mit dem Konnektor der Versorgungsschnittstelle 5 der Konnektoreinheit 3 oder mit der integrierten Schnittstelle 10 der Steuereinheit 2 verbindbar. Die Stromversorgungseinheit kann zum netzgebundenen Betreiben der Steuereinheit 2 und/oder zum Laden des externen Akkus 19 und/oder des internen Akkus eingerichtet sein.

Alternativ kann der zweite Konnektor 15 nur mit der Versorgungsschnittstelle 5, aber nicht mit der integrierten Schnittstelle 10 verbindbar sein. In diesem Fall kann zur direkten Spannungsversorgung der Steuereinheit 2 (ohne Konnektoreinheit 3) eine Standalone-Stromversorgungseinheit vorgesehen sein.

Die Datenschnittstelle 7 ist eine USB-Schnittstelle, kann jedoch auch eine andere Art von Datenschnittstelle sein. Die Konnektoreinheit 3 umfasst eine Schnittstellen-Controller-Schaltung, in diesem Beispiel eine USB-Controller-Schaltung. Die Schnittstellen-Controller-Schaltung kann alternativ ein Teil der Steuereinheit 2 sein; es kann dann vorgesehen sein, dass die Konnektoreinheit 3 keine eigene Schnittstellenlogik umfasst.

Die Konnektoreinheit 3 weist zusätzlich zu der drahtgebundenen Datenschnittstelle 7 eine drahtlose Datenschnittstelle auf, vorzugsweise eine Bluetooth-Schnittstelle. Mittels dieser kann zusätzlich oder alternativ zu der drahtgebundenen Datenverbindung eine drahtlose Datenverbindung der Steuereinheit 2 mit der Datenverarbeitungseinheit 8 hergestellt werden, ohne dass die Steuereinheit 2 selbst eine drahtlose Datenschnittstelle umfasst. Die drahtlose Datenschnittstelle kann alternativ weggelassen werden. Auch kann die Steuereinheit 2 alternativ eine drahtlose Datenschnittstelle umfassen.

Die Steuereinheit 2 des Steuersystems 1 kann gemeinsam mit der Konnektoreinheit des Steuersystems in einer Sterilverpackung versiegelt sein, womit das Steuersystem 1 in einem Zustand bereitstellbar und lagerbar ist, der einen schnellen und einfachen Einsatz etwa im sterilen Operationsbereich 11 ermöglicht. Dabei ist vorgesehen, dass die Steuereinheit 2 ohne den externen Akku 19 und internen Akku in der Sterilverpackung versiegelt ist.

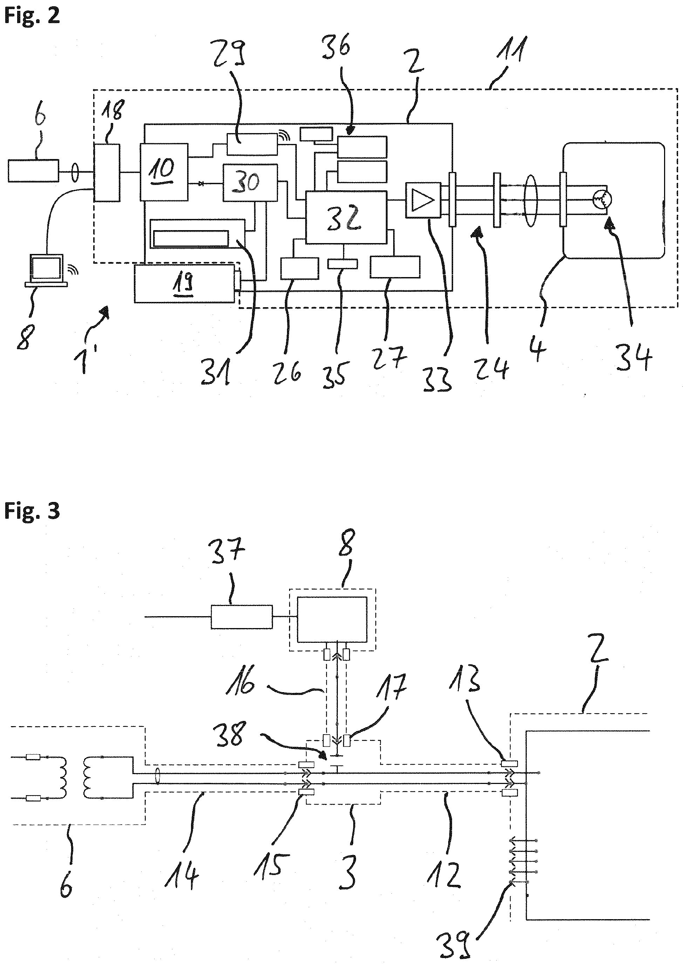

Das in Fig. 2 gezeigte Steuersystem 1' gleicht dem in Fig. 1 gezeigten Steuersystem 1 in weiten Teilen, weshalb hier nur auf Unterschiede und zusätzlich gezeigte Details (die auch bei dem Steuersystem 1 oder 1" vorgesehen sein können) eingegangen wird, wobei diese Unterschiede und Details optionale Merkmale eines Steuersystems der vorgeschlagenen Art darstellen. Insbesondere illustriert Fig. 2 die Architektur einer beispielhaften Steuereinheit 2.

Im Beispiel des Steuersystems 1' umfasst die Steuereinheit 2 eine Schnittstellen-Controller-Schaltung 29. Die Schnittstellen-Controller-Schaltung 29 umfasst eine UART-Controller-Schaltung zum Herstellen der drahtgebundenen Datenverbindung sowie eine Bluetooth-Controller-Schaltung zum Herstellen einer drahtlosen Datenverbindung mit der Datenverarbeitungseinheit 8. Die Konnektoreinheit 3 umfasst in diesem Fall eine USB-Controller-Schaltung und ist dazu eingerichtet, Kommunikationsdaten zwischen einem USB-Kommunikationsprotokoll zur Kommunikation mit der Datenverarbeitungseinheit 8 und einem UART-Komunikationsprotokoll zur Kommunikation mit der Steuerschnittstelle 2 umzuwandeln. Es kann auch die Verwendung anderer Kommunikationsprotokolle, beispielsweise CAN, RS485, SPI etc. vorgesehen sein.

Die Steuereinheit 2 des Steuersystems 1' umfasst einen Hauptcontroller 32, eingerichtet zum Ansteuern verschiedener Komponenten der Steuereinheit 2, sowie eine Motorsteuerung 33, eingerichtet zum Ansteuern eines Pumpenmotors 34 der Blutpumpe 4.

Ferner umfasst die Steuereinheit 2 eine Power-Management-Schaltung 30, eingerichtet zum Steuern einer Spannungsversorgung des Hauptcontrollers 32, der Blutpumpe 4 und anderer Komponenten des Steuersystems 1. Die Power-Management-Schaltung 30 ist ferner eingerichtet zum Steuern eines Ladens und/oder Entladens des internen Akkus 31 und des externen Akkus 19.

Die Steuereinheit 2 umfasst weiterhin eine Erweiterungsschnittstelle 35, mittels derer die Steuereinheit 2 mit einer Erweiterungseinheit verbindbar ist. Die Erweiterungseinheit kann beispielsweise eine Speichererweiterung, insbesondere eine Speicherkarte, sein. Vorzugsweise ist die Erweiterungsschnittstelle 35 verschließbar, insbesondere wasserdicht verschließbar. Die Erweiterungsschnittstelle 35 kann beispielweise innerhalb der Wartungsschnittstelle zur Aufnahme des internen Akkus 31 angeordnet sein, so dass die Erweiterungsschnittstelle 35 gemeinsam mit der Wartungsschnittstelle verschließbar ist.

Die Steuereinheit kann optional weitere Komponenten 36 umfassen, beispielsweise einen oder mehrere Sensoren (zum Erfassen eines Betriebszustands der Steuereinheit 2 und/oder der Blutpumpe 4) und/oder Alarme (zum Ausgeben einer Benachrichtigung über einen Betriebszustand der Steuereinheit 2 und/oder der Blutpumpe 4).

Das in Fig. 3 teilweise gezeigte Steuersystem 1" gleicht dem in Fig. 1 gezeigten Steuersystem 1 in weiten Teilen, weshalb hier nur auf Unterschiede und zusätzlich gezeigte Details (die auch bei dem Steuersystem 1' oder 1" vorgesehen sein können) eingegangen wird, wobei diese Unterschiede und Details optionale Merkmale eines Steuersystems der vorgeschlagenen Art darstellen. Insbesondere illustriert Fig. 3 Aspekte des Steuersystems 1", die der elektrischen Sicherheit dienen.

Die Konnektoreinheit 3 des Steuersystems 1" umfasst eine Isolationsbarriere 38, realisiert als Luft- und/oder Kriechstrecke zwischen Leiterelementen auf einer Platine der Konnektoreinheit 3. Die Isolationsbarriere ist zum elektrischen Entkoppeln der Steuereinheit 2 von der Datenverarbeitungseinheit 8, insbesondere von einem Netzteil 27 oder einer sonstigen Spannungsversorgung der Datenverarbeitungseinheit 8, eingerichtet. Vorzugsweise ist vorgesehen, dass die Isolationsbarriere den Anforderungen der Norm IEC 60601-1 in der zum Anmeldetag geltenden Fassung (oder einer anderen Norm für die Sicherheit medizinischer Geräte) genügt.

An der Steuereinheit 2 angeordnete Kontakte 39 zum Verbinden mit dem externen Akku sind elektrisch isolierbar und somit spannungsfrei schaltbar. Die Steuereinheit 2 ist dazu eingerichtet, die Kontakte 39 beim Entfernen des externen Akkus automatisch spannungsfrei zu schalten.

### Liste der Bezugszeichen

- 1, 1', 1": Steuersystem,
- 2: Steuereinheit,
- 3: Konnektoreinheit,
- 4: Blutpumpe,
- 5: Versorgungsschnittstelle,
- 6: Stromversorgung,
- 7: Datenschnittstelle,
- 8: Datenverarbeitungseinheit,
- 9: Steuerschnittstelle,
- 10: integrierte Schnittstelle,
- 11: steriler Operationsbereich,
- 12: erstes Verbindungskabel,
- 13: erster Konnektor,
- 14: zweites Verbindungskabel,
- 15: zweiter Konnektor,
- 16: drittes Verbindungskabel,
- 17: dritter Konnektor,
- 18: Gehäuse,
- 19: externer Akku,
- 20: Einschubschiene,
- 21: Wartungsschnittstelle,
- 22: Driveline-Schnittstelle,
- 23: vierter Konnektor,
- 24: Driveline,
- 25: Kupplung,
- 26: Anzeige,
- 27: Bedienfeld,
- 28: Akkuschnittstelle,
- 29: Schnittstellen-Controller-Schaltung,
- 30: Power-Management-Schaltung,
- 31: interner Akku,
- 32: Hauptcontroller,
- 33: Motorsteuerung,
- 34: Pumpenmotor,
- 35: Erweiterungsschnittstelle,
- 36: weitere Komponenten,
- 37: Netzteil,
- 38: Isolationsbarriere,
- 39: Kontakte.

## Patentansprüche

1. Konnektoreinheit (3) für eine zum Ansteuern einer implantierbaren Blutpumpe (4) eingerichtete Steuereinheit (2), wobei die Konnektoreinheit (3) umfasst:
eine Versorgungsschnittstelle (5), eingerichtet zum Verbinden der Konnektoreinheit (3) mit einer Stromversorgung (6),
eine Datenschnittstelle (7), eingerichtet zum Verbinden der Konnektoreinheit (3) mit einer Datenverarbeitungseinheit (8),
eine Steuerschnittstelle (9), eingerichtet zum Verbinden der Konnektoreinheit (3) mit einer integrierten Schnittstelle (10) der Steuereinheit (2), so dass zugleich eine Versorgungsverbindung der Steuereinheit (2) mit der Stromversorgung (6) und eine Datenverbindung der Steuereinheit (2) mit der Datenverarbeitungseinheit (8) mittels der Konnektoreinheit (3) herstellbar sind.

2. Konnektoreinheit (3) nach Anspruch 1, ferner umfassend ein Verbindungskabel (12), wobei die Steuerschnittstelle (9) fest oder lösbar mit einem ersten Ende des Verbindungskabels (12) verbindbar oder verbunden ist und ein zweites Ende des Verbindungskabels (12) einen mit der integrierten Schnittstelle (10) der Steuereinheit (2) zum Herstellen der Versorgungsverbindung und der Stromverbindung verbindbaren integrierten Konnektor (13) aufweist.

3. Konnektoreinheit (3) nach Anspruch 2, wobei der integrierte Konnektor (13) des Verbindungskabels (12) wasserdicht mit der integrierten Schnittstelle (10) der Steuereinheit (2) verbindbar ist.

4. Konnektoreinheit (3) nach einem der vorhergehenden Ansprüche, wobei die Konnektoreinheit (3) sterilisierbar ist und/oder das Verbindungskabel (12) sterilisierbar ist.

5. Konnektoreinheit (3) nach einem der vorhergehenden Ansprüche, wobei die Konnektoreinheit (3) eine USB-Controller-Schaltung (29) umfasst und die Datenschnittstelle (7) eine USB-Schnittstelle ist oder umfasst.

6. Konnektoreinheit (3) nach einem der vorhergehenden Ansprüche, wobei die Konnektoreinheit (3) eine Isolationsbarriere (38), insbesondere eine Luftstrecke und/oder Kriechstrecke, zum elektrischen Entkoppeln der Steuereinheit (2) von der Datenverarbeitungseinheit (8) umfasst.

7. Steuereinheit (2) zum Ansteuern einer implantierbaren Blutpumpe (4), umfassend eine integrierte Schnittstelle (10), die mit der Steuerschnittstelle (9) einer Konnektoreinheit (3) nach einem der Ansprüche 1 bis 6 zum Herstellen der Versorgungsverbindung und der Datenverbindung verbindbar ist.

8. Steuereinheit (2) nach Anspruch 7, eingerichtet zum einteiligen Verbinden mit mindestens einem externen Akku (19), d. h. zum Verbinden mit dem mindestens einen externen Akku (19) ohne zwischengeschaltete Kabelverbindung, insbesondere zum formschlüssigen und/oder kraftschlüssigen und/oder wasserdichten einteiligen Verbinden mit dem mindestens einen externen Akku (19).

9. Steuereinheit (2) nach Anspruch 8, wobei an der Steuereinheit (2) angeordnete Kontakte (39) zum Verbinden mit dem externen Akku (19) spannungsfrei schaltbar und/oder elektrisch isolierbar sind.

10. Steuereinheit (2) nach einem der Ansprüche 7 bis 9, wobei die Steuereinheit (2) sterilisierbar ist.

11. Steuereinheit (2) nach einem der Ansprüche 7 bis 10, wobei die Steuereinheit (2) eine extrakorporale Steuereinheit (2) ist und zum Ansteuern der Blutpumpe (4) mittels einer Driveline (24), die eine reversibel verbindbare Kupplung (25) umfasst, mit der Blutpumpe (4) verbindbar ist.

12. Steuereinheit (2) nach einem der Ansprüche 7 bis 11, wobei die Steuereinheit (2) außer den folgenden vier externen drahtgebundenen elektrischen Schnittstellen keine weitere externe drahtgebundene elektrische Schnittstelle aufweist: die vorgenannte integrierte Schnittstelle (10), eine Driveline-Schnittstelle (22) zum Verbinden der Steuereinheit (2) mit der Blutpumpe (4), eine Akkuschnittstelle (28) zum Verbinden der Steuereinheit (2) mit einem externen Akku (19) und entweder eine verschließbare, vorzugsweise wasserdicht verschließbare, Wartungsschnittstelle (21) zum Einsetzen eines internen Akkus (31) oder eine zusätzliche Akkuschnittstelle zum Verbinden der Steuereinheit (2) mit einem zusätzlichen externen Akku.

13. Steuersystem für eine implantierbare Blutpumpe (4), umfassend:
eine Konnektoreinheit (3) nach einem der Ansprüche 1 bis 6 und
eine Steuereinheit (2) nach einem der Ansprüche 7 bis 12.

14. Steuersystem nach Anspruch 13, ferner umfassend eine Stromversorgungseinheit (6), die wahlweise direkt oder mittels der Konnektoreinheit (3) mit der integrierten Schnittstelle (10) der Steuereinheit (2) verbindbar ist.

15. Steuersystem nach Anspruch 13 oder 14, wobei die Steuereinheit (2) gemeinsam mit der Konnektoreinheit (3) in einer Sterilverpackung versiegelt ist.
